# EUROPEAN PATENT SPECIFICATION

(11) **EP 1 629 778 B1**
(45) Date of publication and mention of the grant of the patent: **17.10.2007**
(21) Application number: 05018095.9
(22) Date of filing: 19.08.2005
(51) Int. Cl.: A61B 8/12, A61B 8/00, A61B 8/14

(54) **Ultrasonic endoscope**
Ultraschall-Endoskop
Endoscope à ultrasons

(30) Priority: 25.08.2004 JP 2004244908
(43) Date of publication of application: 01.03.2006
(73) Proprietor: FUJIFILM Corporation, Minato-ku Tokyo (JP)
(72) Inventor: Satoh, Yoshiaki c/o Fuji Photo Film Co., Ltd., Kanagawa 258-8538 (JP); Tsujita, Kazuhiro c/o Fuji Photo Film Co., Ltd., Kanagawa 258-8538 (JP)
(74) Representative: Klunker . Schmitt-Nilson . Hirsch

(56) References cited:
- US-A- 5 320 104
- US-A- 5 685 311
- US-A- 6 059 731
- US-B1- 6 419 633
- US-B1- 6 461 304

## Description

### BACKGROUND OF THE INVENTION

### Field of the Invention

The present invention relates to an ultrasonic endoscope according to the preamble of claim 1 and an ultrasonic endoscopic apparatus having such an ultrasonic endoscope.

### Description of a Related Art

Conventionally, puncture treatment using an ultrasonic endoscope has been performed while confirming in real time the protrusion state of a puncture needle protruded from the tip of the insertion part of the ultrasonic endoscope inserted into a body on ultrasonic tomographic images obtained by using plural ultrasonic transducers (ultrasonic vibrators) provided at the tip of the insertion part of the ultrasonic endoscope. However, such puncture treatment using the ultrasonic endoscope is so difficult that it requires advanced techniques.

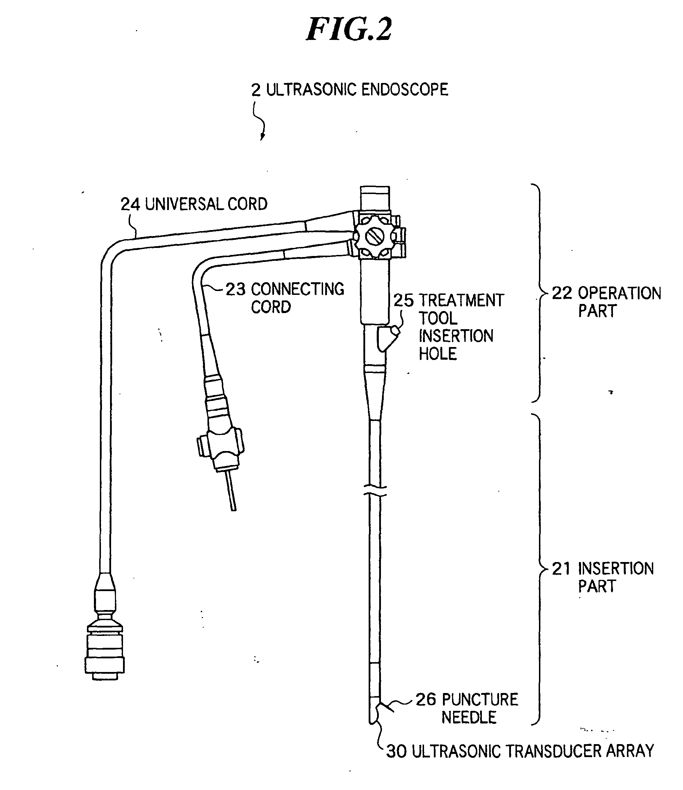

For example, Japanese Patent Application Publication JP-P2004-105289A discloses an ultrasonic endoscope that reduces the difficulty of puncture treatment by suppressing the reaction when the puncture needle is punctured into a target part even if the target part is a tissue that has become hard due to fibrosis or the like (paragraphs 0022 and 0023, and Fig. 2). In the ultrasonic endoscope, a magnetic force is applied from a magnetic generation device located outside of the body of a patient and a current is allowed to flow in an electromagnet provided in a position close to the curved part at the tip side of the flexible portion of the ultrasonic endoscope, and thereby, the position close to the curved part at the tip side of the flexible portion of the ultrasonic endoscope is attracted by a magnetic force and fixed to the luminal wall of the trachea of the patient. Thus, when the puncture needle is punctured into a target part within the body cavity by using the ultrasonic endoscope, the insertion part of the ultrasonic endoscope is prevented from bending due to the reaction of the puncture needle and the puncture needle can be punctured into the target part easily and reliably even if the target part is a tissue that has become hard due to fibrosis or the like.

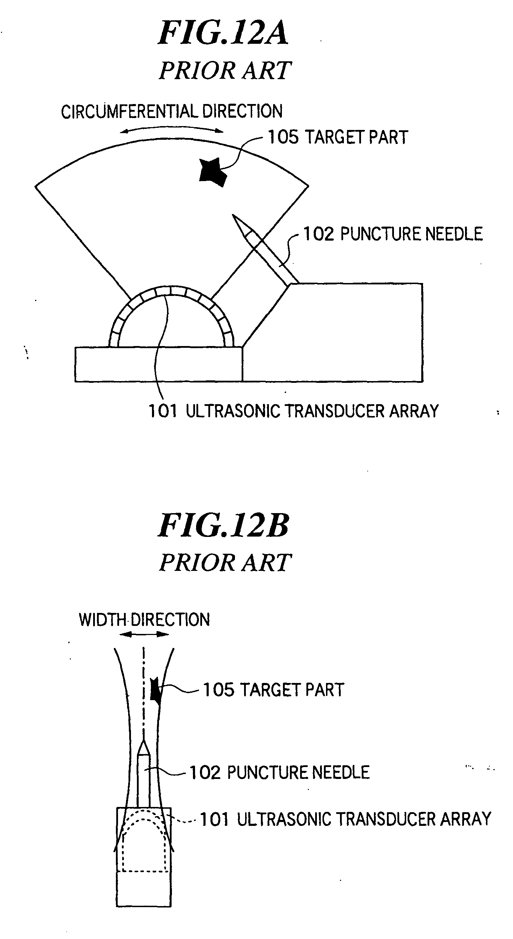

However, a conventional ultrasonic endoscope images ultrasonic tomographic images by using a convex one-dimensional ultrasonic transducer array 101 including plural ultrasonic transducers arranged in a semicircle form as shown in Fig. 12A. As a result, even though it is seen that a puncture needle 102 has been able to be punctured into a target part 105 in a B-mode image, sometimes the center line of the puncture needle 102 and the target part 105 are actually misaligned in the width direction of the ultrasonic transducer because of the focus width in the width direction of the ultrasonic transducer.

Accordingly, in the case where the target part 105 has become hardened, the reaction when the puncture needle 102 is punctured can be suppressed as disclosed in JP-P2004-105289A, however, even in the case where the target part 105 has not become hardened, especially when the target part 105 is small, the difficulty of puncture treatment can not be reduced unless the misalignment between the center line of the puncture needle 102 and the target part 105 because of the focus width in the width direction of the ultrasonic transducer is eliminated.

An ultrasonic endoscope according to the preamble of claim 1 is known from US-A-6,059,731. Specifically, this reference discloses a simultaneous side-and-end viewing ultrasound imaging catheter system including at least one side array and at least one end array. In one embodiment, electrical conductors might directly connect to the ultrasonic transducer array. In an alternative embodiment, the electrical conductors might connect to the ultrasonic transducer array via a functional unit such as an amplifier etc.

### SUMMARY OF THE INVENTION

The present invention is achieved in view of the above-mentioned problems. An object of the present invention is to provide an ultrasonic endoscope and an ultrasonic endoscopic apparatus by which a positional relationship can be grasped even in the width direction of the ultrasonic transducer.

In order to solve the above-mentioned problems, an ultrasonic endoscope according to the present invention comprises the features of claim 1.

An ultrasonic endoscopic apparatus according to the present invention comprises the features of any of claims 3 to 9.

According to the present invention, the positional relationship can be grasped even in the width direction of the ultrasonic transducer, and therefore, the centerline of the puncture needle can be precisely aligned with the target part even when the target part is small, for example.

Further, the number of interconnections within the ultrasonic transducer array can be drastically reduced. As a result, when the ultrasonic transducer array is formed by arranging plural two-dimensional ultrasonic transducer arrays in a semicircle form or polygonal form, the insertion part of the ultrasonic endoscope can be prevented from being thicker due to interconnections within the ultrasonic transducer array.

### BRIEF DESCRIPTION OF THE DRAWINGS

Fig. 1 shows a constitution of an ultrasonic endoscopic apparatus 1 according to a first embodiment of the present invention;
Fig. 2 shows a structure of an ultrasonic endoscope 2 shown in Fig. 1;
Figs. 3A and 3B show a structure of an ultrasonic transducer array 30 shown in Fig. 2;
Fig. 4 shows the structure of the ultrasonic transducer array 30 shown in Fig. 2;
Fig. 5 shows interconnections within the ultrasonic transducer array 30 shown in Fig. 2;
Fig. 6 is a chart for explanation of a method of generating drive pulse signals to be applied to individual electrodes of the respective ultrasonic transducers that form the ultrasonic transducer array 30 shown in Fig. 2;
Fig. 7 is a chart for explanation of a method of generating drive pulse signals to be applied to individual electrodes of the respective ultrasonic transducers that form the ultrasonic transducer array 30 shown in Fig. 2;
Fig. 8 is a diagram for explanation of sector scan with respect to the width direction of the ultrasonic transducer;
Fig. 9 shows a constitution of an ultrasonic endoscopic apparatus 200 according to a second embodiment of the present invention;
Fig. 10 shows interconnections within the ultrasonic transducer array 30 of the ultrasonic endoscopic apparatus 200 shown in Fig. 9;
Figs. 11A to 11D are diagrams for explanation of operation of variable delay line units shown in Fig. 10; and
Figs. 12A and 12B are diagrams for explanation of aproblem in a conventional ultrasonic endoscopic apparatus.

### DESCRIPTION OF THE PREFERRED EMBODIMENTS

Hereinafter, the best modes for implementing the present invention will be described in detail by referring to the drawings . The same reference numbers will be assigned to the same component elements and the description thereof will be omitted.

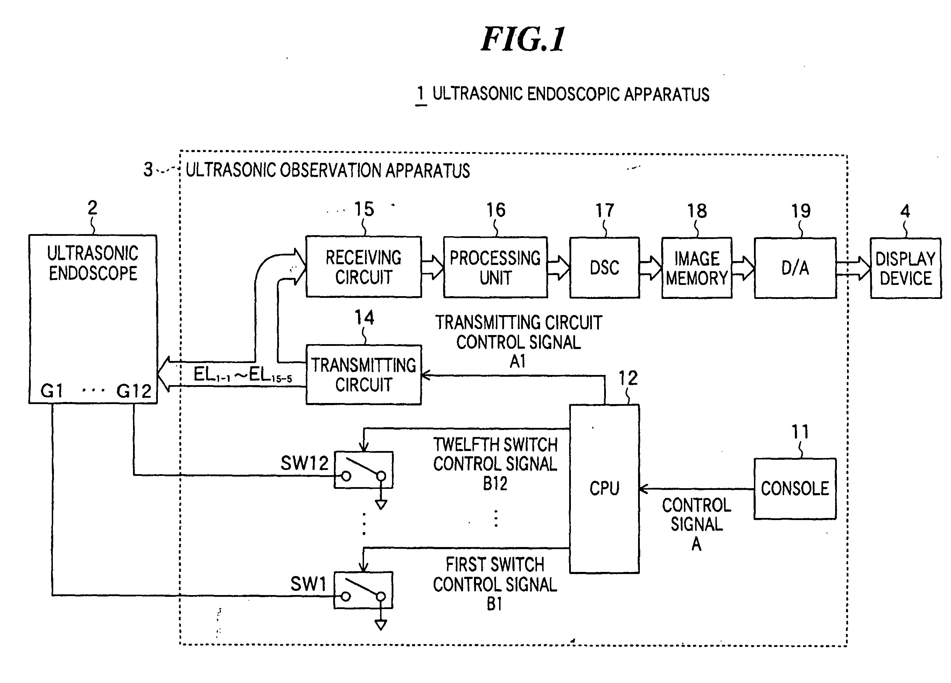

As shown in Fig. 1, an ultrasonic endoscopic apparatus 1 according to a first embodiment of the present invention includes an ultrasonic endoscope 2, an ultrasonic observation apparatus 3 to which the ultrasonic endoscope 2 is connectable, and a display device 4 connected to the ultrasonic observation apparatus 3.

The ultrasonic observation apparatus 3 includes a console 11, aCPU (central processing unit) 12, firsttotwelfth switches SW1 and SW12, a transmitting circuit 14, a receiving circuit 15, a processing unit 16, a digital scan converter (DSC) 17, an image memory 18, and a digital/analog converter (D/A converter) 19.

As shown in Fig. 2, the ultrasonic endoscope 2 includes an insertion part 21, an operation part 22, a connecting cord 23, and a universal cord 24.

The insertion part 21 of the ultrasonic endoscope 2 has an elongated flexible tubular shape so as to be inserted into a body of a patient. The operation part 22 is provided at the base end of the insertion part 21, connected to the ultrasonic observation apparatus 3 via the connecting cord 23 and connected to a light source device and an optical observation device (not shown) via the universal cord 24.

In the insertion part 21 of the ultrasonic endoscope 2, an illumination window and an observation window are provided. An illumination lens for outputting illumination light supplied via a light guide from the light source device is attached to the illumination window. These form an illumination optical system. Further, an objective lens is attached to the observation window, and, in a position where the objective lens forms an image, an input end of an image guide or solid-state image sensor such as a CCD camera is disposed. These form an observation optical system.

Further, at the tip of the insertion part 21 of the ultrasonic endoscope 2, a convex ultrasonic transducer array 30 is provided. The ultrasonic transducer array 30 generates ultrasonic waves according to drive signals supplied from the transmitting circuit 14 of the ultrasonic observation apparatus 3, receives ultrasonic waves reflected from a target part or the like, and outputs plural reception signals to the receiving circuit 15 of the ultrasonic observation apparatus 3. Further, at the tip of the insertion part 21 of the ultrasonic endoscope 2, a hole from which a puncture needle 26 inserted from a treatment tool insertion hole 25 provided in the operation part 22 is protruded is formed.

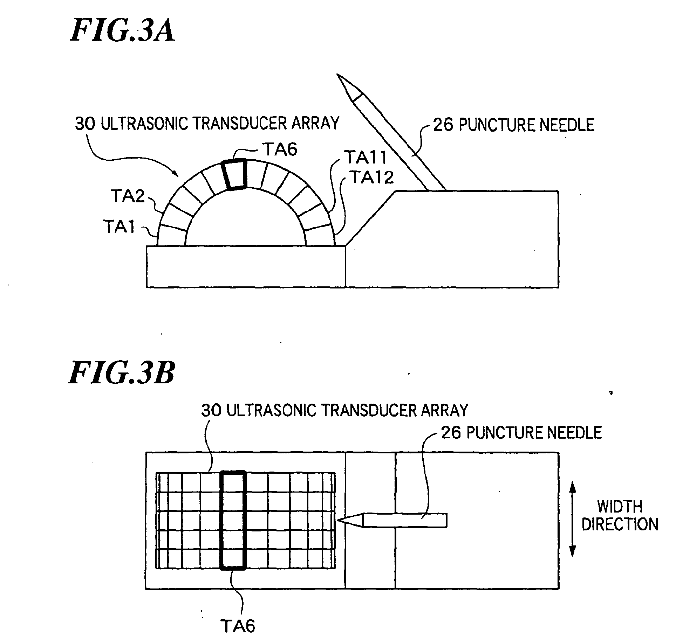

As shown in Figs. 3A and 3B, the ultrasonic transducer array 30 is formed by arranging first to twelfth two-dimensional ultrasonic transducer arrays TA1 to TA12 in a semicircle form for three-dimensional scan with respect to each of the first to twelfth two-dimensional ultrasonic transducer arrays TA1 to TA12.

Here, each of the first to twelfth two-dimensional ultrasonic transducer arrays TA1 to TA12 is formed by arranging ultrasonic transducers in 15 rows in the circumferential direction of the ultrasonic transducer array 30 and five columns in the width direction thereof.

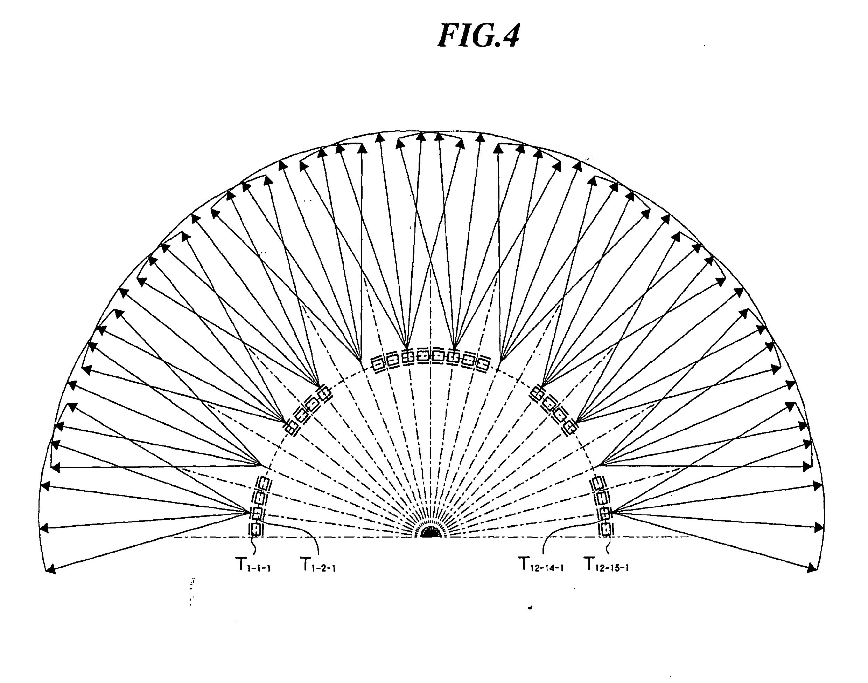

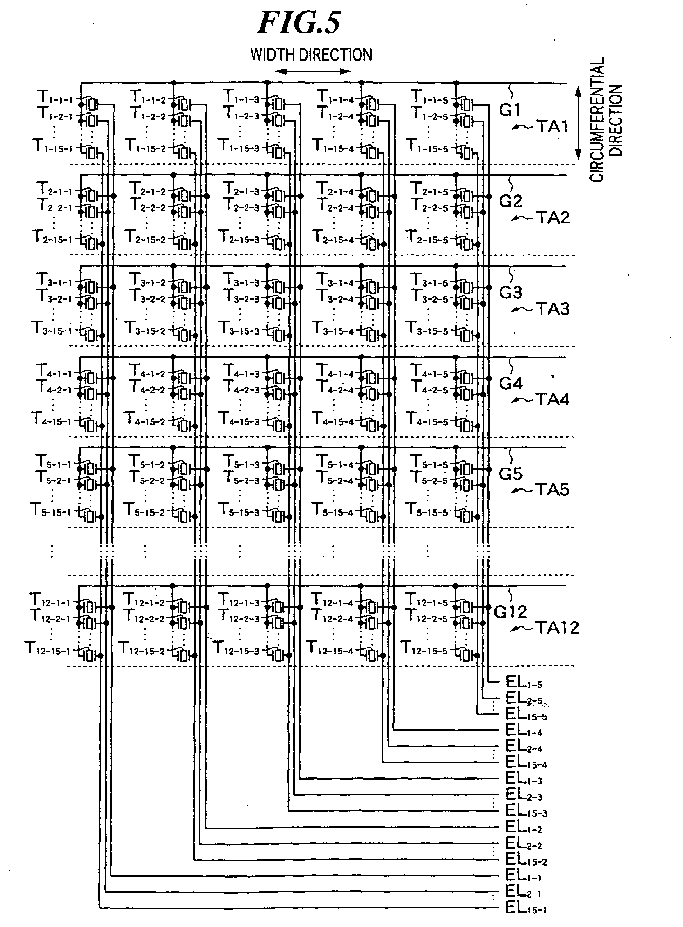

For example, as shown in Figs. 4 and 5, the first two-dimensional ultrasonic transducer array TA1 consists of 15 ultrasonic transducers T₁₋₁₋₁ to T₁₋₁₅₋₁ in the first column, 15 ultrasonic transducers T₁₋₁₋₂ to T₁₋₁₅₋₂ in the second column, 15 ultrasonic transducers T₁₋₁₋₃ to T₁₋₁₅₋₃ in the third column, 15 ultrasonic transducers T₁₋₁₋₄ to T₁₋₁₅₋₄ in the fourth column, and 15 ultrasonic transducers T₁₋₁₋₅ to T₁₋₁₅₋₅ in the fifth column. Other two-dimensional ultrasonic transducer arrays TA2 to TA12 similarly consist of the ultrasonic transducers.

Note that, in the ultrasonic imaging field, an ultrasonic transducer array including different numbers of ultrasonic transducers in rows and columns is generally called "1.5-dimensional ultrasonic transducer array" and an ultrasonic transducer array including the same number of ultrasonic transducers in rows and columns is generally called "two-dimensional ultrasonic transducer array", however, in the specification, both are referred to as "two-dimensional ultrasonic transducer array".

Each of the ultrasonic transducers T₁₋₁₋₁ to T₁₂₋₁₅₋₅ includes an ultrasonic vibrator formed by sandwiching a piezoelectric element of PZT, PVDF, or the like between an individual electrode and a common electrode. When a drive signal is applied to the individual electrode while the common electrode is connected to a fixed potential (for example, a ground potential in the embodiment), the transducer transmits ultrasonic wave, and receives ultrasonic waves reflected from a target part or the like and generates a reception signal in the individual electrode.

In the first to twelfth two-dimensional ultrasonic transducer arrays TA1 to TA12, the individual electrodes of the ultrasonic transducers located in the same rows and columns are electrically connected to the same signal interconnections EL₁₋₁ to EL₁₅₋₅, respectively, as shown in Fig. 5.

For example, in the first to twelfth two-dimensional ultrasonic transducer arrays TA1 to TA12, the individual electrodes of the 12 ultrasonic transducers T₁₋₁₋₁, T₂₋₁₋₁, T₃₋₁₋₁, T₄₋₁₋₁, T₅₋₁₋₁, T₆₋₁₋₁, T₇₋₁₋₁, T₈₋₁₋₁, T₉₋₁₋₁, T₁₀₋₁₋₁, T₁₁₋₁₋₁, and T₁₂₋₁₋₁ located in the first row and the first column are electrically connected to the signal interconnection EL₁₋₁. Further, in the first to twelfth two-dimensional ultrasonic transducer arrays TA1 to TA12, the individual electrodes of the 12 ultrasonic transducers T₁₋₂₋₁, T₂₋₂₋₁, T₃₋₂₋₁, T₄₋₂₋₁, T₅₋₂₋₁, T₆₋₂₋₁, T₇₋₂₋₁, T₈₋₂₋₁, T₉₋₂₋₁, T₁₀₋₂₋₁, T₁₁₋₂₋₁, and T₁₂₋₂₋₁ located in the second row and the first column are electrically connected to the signal interconnection EL₂₋₁.

Further, the common electrodes of the ultrasonic transducers T₁₋₁₋₁ to T₁₂₋₁₅₋₅ that form the first to twelfth two-dimensional ultrasonic transducer arrays TA1 to TA12 are electrically connected to the same common interconnections G1 to G12 with respect to each of the first to twelfth two-dimensional ultrasonic transducer arrays TA1 to TA12 as shown in Fig. 5.

For example, the common electrodes of the 15 × 5 ultrasonic transducers T₁₋₁₋₁ to T₁₋₁₅₋₁, T₁₋₁₋₂ to T₁₋₁₅₋₂, T₁₋₁₋₃ to T₁₋₁₅₋₃, T₁₋₁₋₄ to T₁₋₁₅₋₄, and T₁₋₁₋₅ to T₁₋₁₅₋₅ that form the first two-dimensional ultrasonic transducer array TA1 are electrically connected to the common interconnection G1. Further, the common electrodes of the 15 × 5 ultrasonic transducers T₂₋₁₋₁ to T₂₋₁₅₋₁, T₂₋₁₋₂ to T₂₋₁₅₋₂, T₂₋₁₋₃ to T₂₋₁₅₋₃, T₂₋₁₋₄ to T₂₋₁₅₋₄, and T₂₋₁₋₅ to T₂₋₁₅₋₅ that form the second two-dimensional ultrasonic transducer array TA2 are electrically connected to the common interconnection G2.

Since the individual electrodes and the common electrodes of the ultrasonic transducers T₁₋₁₋₁ to T₁₂₋₁₅₋₅ are thus interconnected, and then, the number of interconnections becomes "87" as the sum of "75" (= 15 × 5) as the number of signal interconnections EL₁₋₁ to EL₁₅₋₅ and "12" as the number of common interconnections G1 to G12. Consequently, the number of interconnections can be drastically reduced compared to the number of interconnections "901" (= 12 × 15 × 5 + 1) in the case where signal interconnections are provided to the individual electrodes of the ultrasonic transducers T₁₋₁₋₁ to T₁₂₋₁₅₋₅, respectively, and one common interconnection is wired to the common electrodes.

The signal interconnections EL₁₋₁ to EL₁₅₋₅ are connected to the transmitting circuit 14 and the receiving circuit 15 of the ultrasonic observation apparatus 3 shown in Fig. 1 via the connecting cord 23 shown in Fig. 2, and the common interconnections G1 to G12 are connected to the first to twelfth switches SW1 to SW12 of the ultrasonic observation apparatus 3 via the connecting cord 23.

The console 11 of the ultrasonic observation apparatus 3 shown in Fig. 1 outputs control signal A for controlling start/stop of ultrasonic imaging operation in the ultrasonic endoscope 2 to the CPU 12.

The CPU 12 outputs first to twelfth switch control signals B1 to B12 for controlling ON/OFF of the first to twelfth switches SW1 to SW12 based on the control signal A input from the console 11 to the first to twelfth switches SW1 to SW12, respectively, and outputs transmitting circuit control signal A1 for controlling the start/stop of operation of the transmitting circuit 14 and delay times (delay amounts) of the drive signals to the transmitting circuit 14.

The first to twelfth switches SW1 to SW12 are turned ON/OFF according to the first to twelfth switch control signals B1 to B12 input from the CPU 12. When the first to twelfth switches SW1 to SW12 are turned ON, the common interconnections G1 to G12 are grounded, respectively, and, when the first to twelfth switches SW1 to SW12 are turned OFF, the common interconnections G1 to G12 are opened, respectively. Since the ultrasonic transducer operates only when the common electrode is grounded, the ultrasonic transducers T₁₋₁₋₁ to T₁₂₋₁₅₋₅ can be operated with respect to each of the first to twelfth two-dimensional ultrasonic transducer arrays TA1 to TA12.

When the transmitting circuit control signal A1 for instructing the operation start of the transmitting circuit 14 is input from the CPU 12, the transmitting circuit 14 generates drive signals and outputs them onto the signal interconnections EL₁₋₁ to EL₁₅₋₅.

The receiving circuit 15 amplifies the reception signals input from the plural ultrasonic transducers with the grounded common electrodes included in the ultrasonic transducer array via the signal interconnections EL₁₋₁ to EL₁₅₋₅ at a predetermined amplification degree, and then, converts the amplified reception signals into digital reception signals by performing A/D conversion. Further, the receiving circuit 15 performs reception focus processing by performing processing of phase matching or the like on the digital reception signals to form sound ray data in which focal points of the ultrasonic echoes are narrowed.

The processing unit 16 performs correction of attenuation depending on the distance according to the depth of the reflection position of ultrasonic wave on the sound ray data formed by the receiving circuit 15 and performs envelope detection processing thereon to generate B-mode image data. Alternatively, the processing unit 16 generates Doppler image data formed by extracting only reflection components from bloodstream based on the sound ray data formed by the receiving circuit 15.

Since the B-mode image data or the Doppler image data generated in the processing unit 16 has been obtained by the scanning method different from the normal scanning method of television signal, the DSC 17 converts (raster conversion) the data into normal image data. The image memory 18 stores the image data generated in the DSC 17. The D/A converter 19 converts the digital image data read from the image memory 18 into analog image signals and outputs them to the display unit 4. Thereby, in the display unit 4, a three-dimensional ultrasonic tomographic image imaged by the ultrasonic endoscope 2 is displayed.

Next, an operation of the ultrasonic endoscopic apparatus 1 according to the embodiment will be described.

In the case where an ultrasonic tomographic image is imaged using the ultrasonic endoscope 2 shown in Fig. 2, the operator emits light from the light source device connected to one end of the universal cord 24 and outputs illumination light into the body of the patient from the illumination window provided at the tip of the insertion part 21, and inserts the insertion part 21 of the ultrasonic endoscope 2 into the body of the patient while observing the insertion state from the observation window.

When the insertion part 21 reaches a target position, the operator allows the console 11 to output the control signal A for starting the operation of the ultrasonic endoscope 2 to the CPU 12 (see Fig. 1). The CPU 12 outputs the first switch control signal B1 for turning ON the first switch SW1 to the first switch SW1 and outputs the transmitting circuit control signal A1 for starting the operation of the transmitting circuit 14 to the transmitting circuit 14 according to the control signal A.

When the first switch SW1 is turned ON, the common interconnection G1 is grounded and the common electrodes of the ultrasonic transducers T₁₋₁₋₁ to T₁₋₁₅₋₅ that form the first two-dimensional ultrasonic transducer array TA1 shown in Fig. 5 are grounded.

The transmitting circuit 14 respectively generates drive signals to be applied to the individual electrodes of the ultrasonic transducers T₁₋₁₋₁ to T₁₋₁₅₋₅ hat form the first two-dimensional ultrasonic transducer array TA1 via the signal interconnections EL₁₋₁ to EL₁₅₋₅. Simultaneously, the transmitting circuit 14 provides delay times to the respective drive signals (pulse signals in the embodiment) based on the transmitting circuit control signal A1 from the CPU 12 in order to perform transmission beam forming and steering of transmission beam.

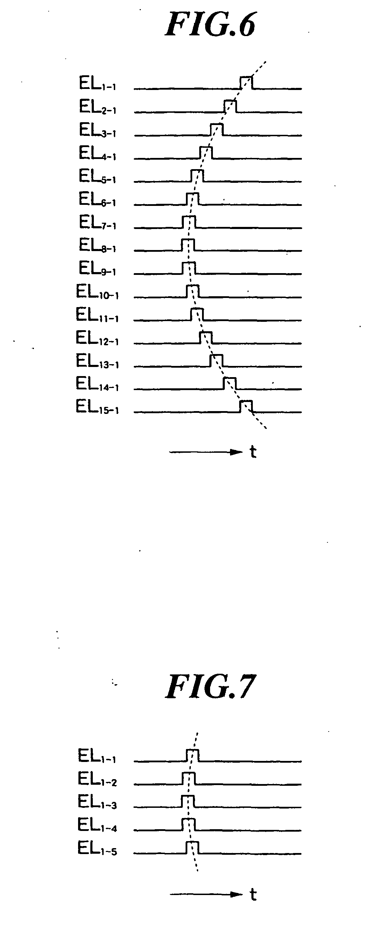

That is, the transmitting circuit 14 provides delay times as shown in Fig. 6 to the drive pulse signals to be applied to the 15 ultrasonic transducers T₁₋₁₋₁ to T₁₋₁₅₋₅ in the first column of the first two-dimensional ultrasonic transducer array TA1 via the signal interconnections EL₁₋₁ to EL₁₅₋₁ in order to perform transmission beam forming with respect to the circumferential direction of the ultrasonic transducer. Further, the transmitting circuit 14 changes the delay times of the drive signals with time such that the sector scan with viewing angle of about 60° is performed with respect to the circumferential direction of the ultrasonic transducer as shown in Fig. 4. The operation is the same for the drive pulse signals to be applied to the ultrasonic transducers in the second to fifth columns of the first two-dimensional ultrasonic transducer array TA1.

Further, the transmitting circuit 14 provides delay times as shown in Fig. 7 to the respective drive pulse signals to be applied to the five ultrasonic transducers T₁₋₁₋₁, T₁₋₁₋₂, T₁₋₁₋₃, T₁₋₁₋₄, and T₁₋₁₋₅ in the first row of the first two-dimensional ultrasonic transducer array TA1 shown in Fig. 5 via the signal interconnections EL₁₋₁, EL₁₋₂, EL₁₋₃, EL₁₋₄, and EL₁₋₅ in order to perform transmission beam forming with respect to the width direction of the ultrasonic transducer. Further, the transmitting circuit 14 changes the delay times of the drive pulse signals with time such that the sector scan with predetermined viewing angle is performed with respect to the width direction of the ultrasonic transducer. The operation is the same for the drive pulse signals to be applied to the ultrasonic transducers in the second to fifteenth rows of the first two-dimensional ultrasonic transducer array TA1.

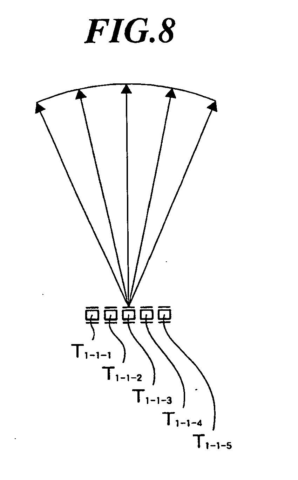

Thereby, ultrasonic waves are emitted from the ultrasonic transducers T₁₋₁₋₁ to T₁₋₁₅₋₅ that form the first two-dimensional ultrasonic transducer array TA1, and the sector scan with viewing angle of about 60° is performed with respect to the circumferential direction of the ultrasonic transducer as shown in Fig. 4 and the sector scan is also performed with respect to the width direction as shown in **Fig. 8.**

The plural reception signals obtained by the sector scan are output to the receiving circuit 15, sound ray data is formed, the B-mode image data or the Doppler image data is generated in the processing unit 16, and then, the data is raster-converted in the DSC 17 and image data is formed. The image data is stored in the image memory 18.

When the sector scan by the first two-dimensional ultrasonic transducer array TA1 is finished, the CPU 12 outputs the first switch control signal B1 for turning OFF the first switch SW1 to the first switch SW1 and outputs the second switch control signal B2 for turning ON the second switch SW2 to the second switch SW2. Thereby, the common interconnection G2 is grounded and the common electrodes of the ultrasonic transducers T₂₋₁₋₁ to T₂₋₁₅₋₅ that forms the second two-dimensional ultrasonic transducer array TA2 are grounded.

In the transmitting circuit 14, the drive pulse signals to be applied to the individual electrodes of the ultrasonic transducers T₂₋₁₋₁ to T₂₋₁₅₋₅ that form the second two-dimensional ultrasonic transducer array TA2 via the signal lines EL₁₋₁ to EL₁₅₋₅ are respectively generated as described above.

Thereby, ultrasonic waves are emitted from the ultrasonic transducers T₂₋₁₋₁ to T₂₋₁₅₋₅ that form the second two-dimensional ultrasonic transducer array TA2, and the sector scan with viewing angle of about 60° is performed with respect to the circumferential direction of the ultrasonic transducer as shown in Fig. 4 and the sector scan is also performed with respect to the width direction as shown in Fig. 8.

The plural reception signals obtained by the sector scan are output to the receiving circuit 15, sound ray data is formed, the B-mode image data or the Doppler image data is generated in the processing unit 16, and then, the data is raster-converted in the DSC 17 and image data is formed. The image data is stored in the image memory 18.

The above operation is repeated, and thereby, sector scan is performed with respect to each of the first to twelfth two-dimensional ultrasonic transducer arrays TA1 to TA12 with respect to the circumferential direction of the ultrasonic transducer while performing sector scan with respect to the width direction of the ultrasonic transducer.

As a result, image data obtained by the three-dimensional scanning with the ultrasonic endoscope 2 is stored in the image memory 18. The image data is read from the image memory 18, and then, converted into analog image signals by the D/A converter 19, and output to the display device 4. Thereby, in the display unit 4, a three-dimensional ultrasonic tomographic image imaged by the ultrasonic endoscope 2 is displayed.

Since the three-dimensional ultrasonic tomographic image is formed also by scanning with respect to the width direction of the ultrasonic transducer, the operator can precisely puncture the puncture needle 26 into the target part by allowing the puncture needle 26 inserted from the treatment tool insertion hole 25 shown in Fig. 2 to protrude from the hole at the tip of the insertion part 21 while confirming the position of the target part in the three-dimensional ultrasonic tomographic image displayed in the display device 4.

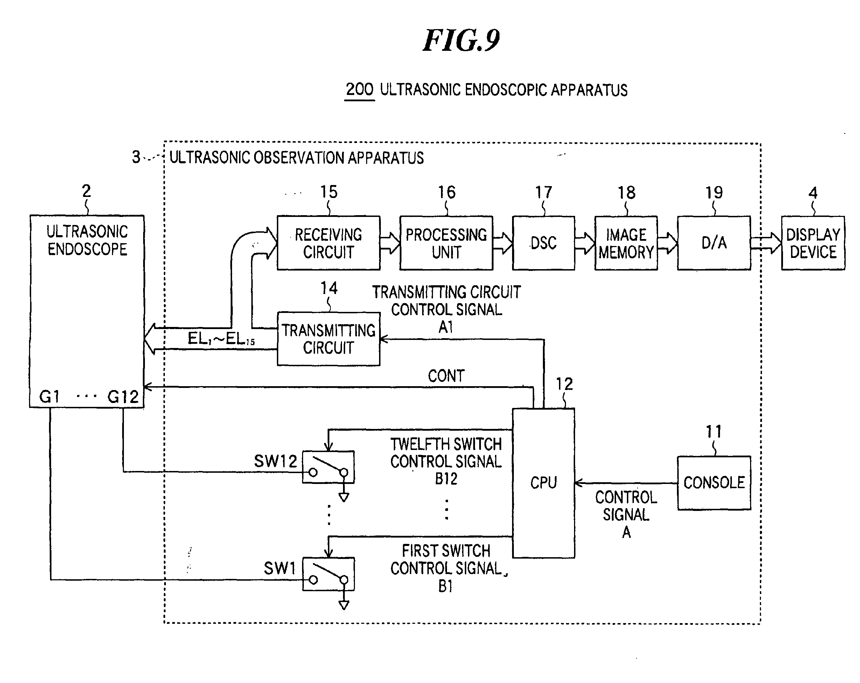

Next, an ultrasonic endoscopic apparatus according to the second embodiment of the present invention will be described by referring to Figs. 9 to 11D.

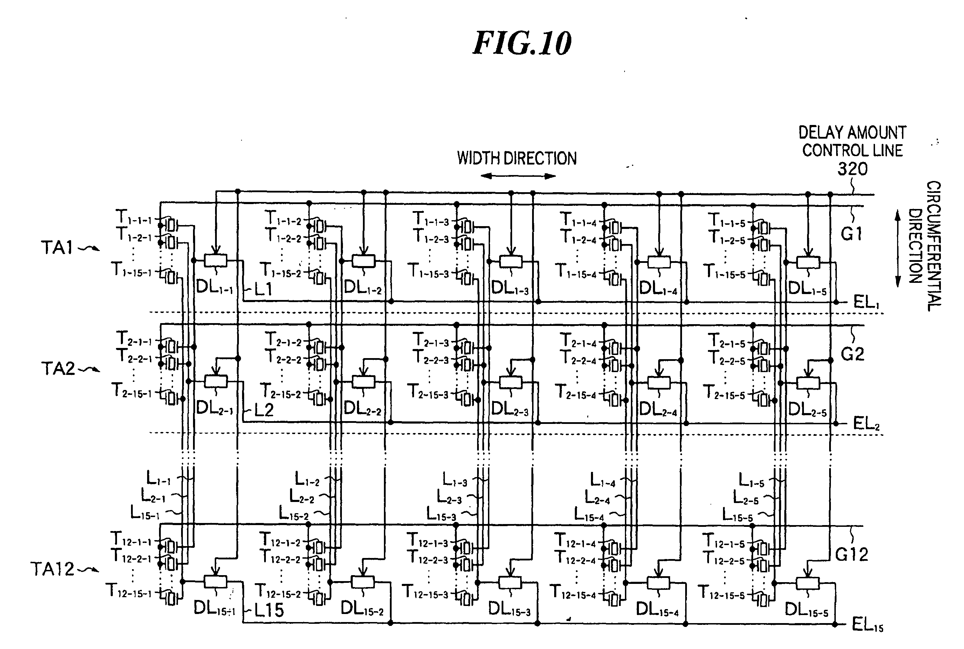

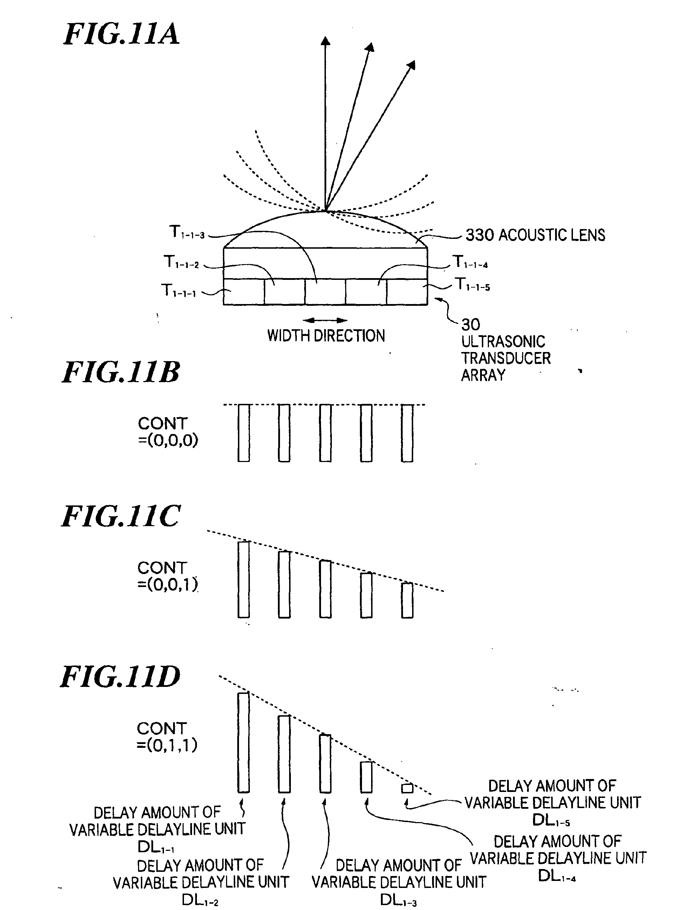

An ultrasonic endoscopic apparatus 200 according to the embodiment differs from the ultrasonic endoscopic apparatus 1 according to the first embodiment shown in Fig. 1 in the following points (1) to (4):
(1) As shown in Fig. 9, the CPU 12 of the ultrasonic observation apparatus 3 outputs a 3-bit delay amount control signal CONT for switching the delay amounts of variable delay line units DL₁₋₁ to DL₁₅₋₅ provided in the ultrasonic endoscope 2, which will be described later, to delay amount control lines 320 (Fig. 10) when the control signal A for instructing the start of the operation of the ultrasonic endoscope 2 from the console 11;
(2) The number of the signal interconnections EL₁ to EL₁₅ output from the transmitting circuit 14 of the ultrasonic observation apparatus 3 is 15;
(3) As shown in Fig. 10, the signal interconnections EL1 to EL15 include plural first conducting lines L₁₋₁ to L₁₅₋₅ to which the individual electrodes of the ultrasonic transducers located in the same rows and columns in the first to twelfth two-dimensional ultrasonic transducer arrays TA1 to TA12 are electrically connected and plural second conducting lines L1 to L15 for inputting the same drive pulse signals to the individual electrodes of the ultrasonic transducers located in the same rows and columns in the first to twelfth two-dimensional ultrasonic transducer arrays TA1 to TA12. Further, the variable delay line units DL₁₋₁ to DL₁₅₋₅ are respectively provided between the plural first conducting lines L₁₋₁ to L₁₅₋₅ and second conducting lines L1 to L15, and the delay amount control lines 320 for inputting the delay amount control signal CONT for controlling the delay amounts of the variable delay line units DL₁₋₁ to DL₁₅₋₅ to the variable delay line units DL₁₋₁ to DL₁₅₋₅ are connected to the variable delay line units DL₁₋₁ to DL₁₅₋₅.
   That is, the same drive pulse signals are input to the individual electrodes of the ultrasonic transducers T₁₋₁₋₁ to T₁₂₋₁₅₋₅ located in the same rows and columns in the first to twelfth two-dimensional ultrasonic transducer arrays TA1 to TA12 via the variable delay line units DL₁₋₁ to DL₁₅₋₅.
   For example, the drive pulse signal output from the transmitting circuit 14 shown in Fig. 9 onto the signal interconnection EL₁ is input to the individual electrodes of the ultrasonic transducers T₁₋₁₋₁, T₂₋₁₋₁, T₃₋₁₋₁, T₄₋₁₋₁, T₅₋₁₋₁, T₆₋₁₋₁, T₇₋₁₋₁, T₈₋₁₋₁, T₉₋₁₋₁, T₁₀₋₁₋₁, T₁₁₋₁₋₁, and T₁₂₋₁₋₁ located in the first row and the first column in the first to twelfth two-dimensional ultrasonic transducer arrays TA1 to TA12 via the first conducting line L₁₋₁, the variable delay line unit DL₁₋₁, and the second conducting line L1. Further, the drive pulse signal output from the transmitting circuit 14 shown in Fig. 9 onto the signal interconnection EL₂ is input to the individual electrodes of the ultrasonic transducers T₁₋₂₋₁, T₂₋₂₋₁, T₃₋₂₋₁, T₄₋₂₋₁, T₅₋₂₋₁, T₆₋₂₋₁, T₇₋₂₋₁, T₈₋₂₋₁, T₉₋₂₋₁, T₁₀₋₂₋₁, T₁₁₋₂₋₁, and T₁₂₋₂₋₁ located in the second row and the first column in the first to twelfth two-dimensional ultrasonic transducer arrays TA1 to TA12 via the first conducting line L₂₋₁, the variable delay line unit DL₂₋₁, and the second conducting line L2.
   Thereby, the number of interconnections within the ultrasonic transducer array 30 becomes 15 (signal interconnections EL₁ to EL₁₅) + 12 (common interconnections G1 to G12) + 3 (delay amount control lines 320) = 30, and the number can be drastically reduced compared to that in the ultrasonic endoscope 2 according to the first embodiment shown in Fig. 5.
   The variable delay line units DL₁₋₁ to DL₁₅₋₅ connected to the same signal interconnections EL₁ to EL₁₅ can change the combination of delay amounts by the 3-bit delay amount control signal CONT input from the CPU 12 via the delay amount control lines 320 among eight different combinations such that the sector scan in the width direction of the ultrasonic transducer can be performed as shown in Fig. 11A, for example.
   When the delay amount control signal CONT (0, 0, 0) is input as shown in Fig. 11B, the variable delay line units DL₁₋₁ to DL₁₅₋₅ connected to the signal interconnection EL₁ are controlled to be provided with the same delay amount. When the delay amount control signal CONT (0, 0, 1) is input as shown in Fig. 11C, the delay amounts are controlled to be smaller by a first amount from the variable delay line unit DL₁₋₁ toward the variable delay line unit DL₁₋₅. When the delay amount control signal CONT (0, 1, 1) is input as shown in Fig. 11D, the delay amounts are controlled to be smaller by a second amount, which is larger than the first amount, from the variable delay line unit DL₁₋₁ toward the variable delay line unit DL₁₋₅.
(4) In the ultrasonic endoscope 2, as shown in Fig. 11A, an acoustic lens 330 for performing transmission beam forming with respect to the width direction of the ultrasonic transducer is provided on the ultrasonic transducer array 30.

Next, an operation of the ultrasonic endoscopic apparatus 200 according to the embodiment will be described, however, because the operation of the receiving circuit 15, the processing unit 16, the DSC 17, the image memory 18, the D/A converter 19, and the display device 4 is the same as the operation in the ultrasonic endoscopic apparatus 1 according to the first embodiment shown in Fig. 1, the description thereof will be omitted.

When the insertion part 21 reaches a target position, the operator allows the console 11 to output the control signal A for starting the operation of the ultrasonic endoscope 2 to the CPU 12. The CPU 12 sequentially outputs one delay amount control signal CONT of (0, 0, 0) to (1, 1, 1) onto the delay amount control lines 320 for sector scan in the width direction of the ultrasonic transducer, outputs the first switch control signal B1 for turning ON the first switch SW1 to the first switch SW1, and outputs the transmitting circuit control signal A1 for starting the operation of the transmitting circuit 14 according to the control signal A.

When the first switch SW1 is turned ON, the common interconnection G1 is grounded and the common electrodes of the ultrasonic transducers T₁₋₁₋₁ to T₁₋₁₅₋₅ that form the first two-dimensional ultrasonic transducer array TA1 are grounded.

The transmitting circuit 14 respectively generates drive signals to be applied to the individual electrodes of the ultrasonic transducers T₁₋₁₋₁ to T₁₋₁₅₋₅ via the signal interconnections EL₁ to EL₁₅. Simultaneously, the transmitting circuit 14 provides predetermined delay times as shown in Fig. 6 to the respective pulse signals in order to perform transmission beam forming with respect to the circumferential direction of the ultrasonic transducer, and changes with time the delay times of the drive pulse signals provided with the predetermined delay time such that the sector scan with viewing angle of about 60° is performed with respect to the circumferential direction of the ultrasonic transducer as shown in Fig. 4. Further, since the transmission beam forming is performed by the acoustic lens 330 (Fig. 11A) with respect to the width direction of the ultrasonic transducer, the transmitting circuit 14 does not provide the predetermined delay times as shown in Fig. 7 to the respective drive pulse signals.

In the case where the acoustic lens 330 is not used, the transmitting circuit 14 may provide the predetermined delay times as shown in Fig. 7 to the respective drive pulse signals in order to perform transmission beam forming with respect to the width direction of the ultrasonic transducer.

Thereby, ultrasonic waves are emitted from the ultrasonic transducers T₁₋₁₋₁ to T₁₋₁₅₋₅ that form the first two-dimensional ultrasonic transducer array TA1, and the sector scan with viewing angle of about 60° is performed with respect to the circumferential direction of the ultrasonic transducer as shown in Fig. 4 and the sector scan is also performed with respect to the width direction as shown in Fig. 8. The reception signals obtained by the sector scan are output to the receiving circuit 15 via the signal interconnections EL₁ to EL₁₅.

When the sector scan by the first two-dimensional ultrasonic transducer array TA1 is finished, the first switch control signal B1 for turning OFF the first switch SW1 is output to the first switch SW1 and the second switch control signal B2 for turning ON the second switch SW2 is output to the second switch SW2. Thereby, the common interconnection G2 is grounded and the common electrodes of the ultrasonic transducers T₂₋₁₋₁ to T₂₋₁₅₋₅ that form the second two-dimensional ultrasonic transducer array TA2 are grounded.

The transmitting circuit 14 respectively generates the drive pulse signals to be applied to the individual electrodes of the ultrasonic transducers T₂₋₁₋₁ to T₂₋₁₅₋₅ that form the second two-dimensional ultrasonic transducer array TA2 via the signal lines EL₁ to EL₁₅ as described above.

Thereby, ultrasonic waves are emitted from the ultrasonic transducers T₂₋₁₋₁ to T₂₋₁₅₋₅ that form the second two-dimensional ultrasonic transducer array TA2, and the sector scan with viewing angle of about 60° is performed with respect to the circumferential direction of the ultrasonic transducer as shown in Fig. 4 and the sector scan is also performed with respect to the width direction as shown in Fig. 8. The plural reception signals obtained by the sector scan are output to the receiving circuit 15 via the signal interconnections EL₁ to EL₁₅.

The above operation is repeated, and thereby, sector scan is performed with respect to each of the first to twelfth two-dimensional ultrasonic transducer arrays TA1 to TA12 with respect to the circumferential direction of the ultrasonic transducer while performing sector scan with respect to the width direction of the ultrasonic transducer.

In the above description, in the ultrasonic endoscope 2, twelve sets of two-dimensional ultrasonic transducer arrays formed by 15 × 5 ultrasonic transducers are arranged in the semicircle form for performing three-dimensional scan with respect to each two-dimensional ultrasonic transducer array, however, these two-dimensional ultrasonic transducer arrays may be arranged in a dodecagonal form for performing three-dimensional scan with respect to each two-dimensional ultrasonic transducer array. Further, the number of ultrasonic transducers that form the two-dimensional ultrasonic transducer array may be not 15 × 5 (including the same number of ultrasonic transducers in the circumferential direction and the width direction).

Further, the first to twelfth switches SW1 and SW12 shown in Figs. 1 and 9 have been provided in the ultrasonic observation apparatus 3, however, they may be provided in the ultrasonic endoscope 2, or provided in an adapter and the ultrasonic endoscope 2 and the observation device 3 may be connected via the adapter.

Furthermore, the control signal A for controlling start/stop of ultrasonic imaging operation of the ultrasonic endoscope 2 has been output from the console 11, however, for example, a button for generating the control signal A may be provided in the operation part 22 of the ultrasonic endoscope 2 and the control signal A may be output from the operation part 22 to the CPU 12.

## Claims

1. An ultrasonic endoscope comprising:
plural two-dimensional ultrasonic transducer arrays (TA1 to TA12) arranged in a semicircle or polygonal form, each including plural ultrasonic transducers arranged in M rows and N columns where M and N are integers not less than 2, for performing three-dimensional scanning operation;
a first group of interconnections (G1 to G12) each for electrically connecting to one another first electrodes of the plural ultrasonic transducers included in respective one of said plural two-dimensional ultrasonic transducer arrays (TA1 to TA12) and electrically connecting a connection point thereof to an external circuit, **characterized by**:
a second group of interconnections (EL₁₋₁ to EL₁₅₋₅, L₁₋₁ to L₁₅₋₅) each for electrically connecting to one another second electrodes of the plural ultrasonic transducers located in a same row and a same column in said plural two-dimensional ultrasonic transducer arrays (TA1 to TA12) and electrically connecting a connection point thereof to an external circuit directly or through a variable delay line unit.

2. An ultrasonic endoscope according to claim 1, further comprising:
plural variable delay line units (DL₁₋₁ to DL₁₅₋₅) having first terminals connected to said second group of interconnections (L₁₋₁ to L₁₅₋₅), respectively; and
a third group of interconnections (L1 to L15) each for electrically connecting to one another second terminals of the plural variable delay line units connected to the plural ultrasonic transducers located in a same row in said plural two-dimensional ultrasonic transducer arrays (TA1 to TA12) and electrically connecting a connection point thereof to an external circuit.

3. An ultrasonic endoscopic apparatus comprising:
an ultrasonic endoscope according to claim 1;
transmitting means (14) for generating drive signals and outputting the drive signals to said plural two-dimensional ultrasonic transducer arrays (TA1 to TA12) via said second group of interconnections (EL₁₋₁ to EL₁₅₋₅) ;
plural pieces of switch means (SW1 to SW12) connected to said first group of interconnections (G1 to G12) and a fixed potential; and
control means (12) for controlling whether or not each of said first group of interconnections (G1 to G12) is connected to the fixed potential by supplying control signals to said plural pieces of switch means (SW1 to SW12) so as to select one to be used from among said plural two-dimensional ultrasonic transducer arrays (TA1 to TA12), and controlling delay amounts of the drive signals in said transmitting means (14) such that the selected two-dimensional ultrasonic transducer array performs the three-dimensional scanning operation within a respective area.

4. An ultrasonic endoscopic apparatus comprising:
an ultrasonic endoscope according to claim 2;
transmitting means (14) for generating drive signals and outputting the drive signals to said plural two-dimensional ultrasonic transducer arrays (TA1 to TA12) via said third group of interconnections (L1 to L15);
plural pieces of switch means (SW1 to SW12) connected to said first group of interconnections (G1 to G12) and a fixed potential; and
control means (12) for controlling whether or not each of saidfirstgroup of interconnections (G1 to G12) is connected to the fixed potential by supplying control signals to said plural pieces of switch means (SW1 to SW12) so as to select one to be used from among said plural two-dimensional ultrasonic transducer arrays (TA1 to TA12), and supplying delay amount control signals for controlling delay amounts of the drive signals to said plural variable delay line units (DL₁₋₁ to DL₁₅₋₅) such that the selected two-dimensional ultrasonic transducer array performs the three-dimensional scanning operation within a respective area.

5. An ultrasonic endoscopic apparatus according to claim 3, said control means (12) controls the delay amounts of the drive signals in said transmitting means (14) so as to perform the three-dimensional scanning operation according to a sector method.

6. An ultrasonic endoscopic apparatus according to claim 4 , said control means (12) supplying the delay amount control signals for controlling delay amounts of the drive signals to said plural variable delay line units (DL₁₋₁ to DL₁₅₋₅) so as to perform the three-dimensional scanning operation according to a sector method.

7. An ultrasonic endoscopic apparatus according to claim 3 or 4, said plural pieces of switch means (SW1 to SW12) are provided within said ultrasonic endoscope.

8. An ultrasonic endoscopic apparatus comprising:
an ultrasonic endoscope according to claim 1;
an adapter including plural pieces of switch means (SW1 to SW12) connected to said first group of interconnections (G1 to G12) and a fixed potential; and
an ultrasonic observation apparatus (3) including transmitting means (14) for generating drive signals and outputting the drive signals to said plural two-dimensional ultrasonic transducer arrays (TA1 to TA12) via said second group of interconnections (EL₁₋₁ to EL₁₅₋₅), and control means (12) for controlling whether or not each of said first group of interconnections (G1 to G12) is connected to the fixed potential by supplying control signals to said plural pieces of switch means (SW1 to SW12) so as to select one to be used from among said plural two-dimensional ultrasonic transducer arrays (TA1 to TA12), and controlling delay amounts of the drive signals in said transmitting means (14) such that the selected two-dimensional ultrasonic transducer array performs the three-dimensional scanning operation within a respective area.

9. An ultrasonic endoscopic apparatus comprising:
an ultrasonic endoscope according to claim 2; and
an adapter including plural pieces of switch means (SW1 to SW12) connected to said first group of interconnections (G1 to G12) and a fixed potential; and
an ultrasonic observation apparatus (3) including transmitting means (14) for generating drive signals and outputting the drive signals to said plural two-dimensional ultrasonic transducer arrays (TA1 to TA12) via said third group of interconnections (L1 to L15), and control means (12) for controlling whether or not each of said first group of interconnections (G1 to G12) is connected to the fixed potential by supplying control signals to said plural pieces of switch means (SW1 to SW12) so as to select one to be used from among said plural two-dimensional ultrasonic transducer arrays (TA1 to TA12), and supplying delay amount control signals for controlling delay amounts of the drive signals to said plural variable delay line units (DL₁₋₁ to DL₁₅₋₅) such that the selected two-dimensional ultrasonic transducer array performs the three-dimensional scanning operation within a respective area.

## Patentansprüche

1. Ultraschallendoskop, umfassend:
mehrere zweidimensionale Ultraschallwandlerfelder (TA1 bis TA12), die in einem Halbkreis oder in Form eines Polygons angeordnet sind, und jeweils mehrere Ultraschallwandler enthalten, die in M Reihen und N Spalten mit M und N als natürliche Zahlen nicht kleiner als 2 angeordnet sind, um einen dreidimensionalen Abtastvorgang durchzuführen;
eine erste Gruppe von Verbindungen (G1 bis G12), jeweils zum elektrischen gegenseitigen Verbinden von ersten Elektroden der mehreren Ultraschallwandler in einem der mehreren zweidimensionalen Ultraschallwandlerfelder (TA1 bis TA12) und zum elektrischen Anschließen eines Anschlußpunkts von diesen mit einer externen Schaltung, **gekennzeichnet durch**:
eine zweite Gruppe von Verbindungen (EL₁₋₁ bis EL₁₅₋₅, L₁₋₁ bis L₁₅₋₅), jeweils zum elektrischen gegenseitigen Verbinden von zweiten Elektroden der mehreren UItraschallwandler, die in der gleichen Reihe und der gleichen Spalte der mehreren zweidimensionalen Ultraschallwandlerfelder (TA1 bis TA12) enthalten sind, und zum elektrischen Verbinden eines Verbindungspunkts von ihnen mit einer externen Schaltung direkt oder über eine variable Verzögerungsleitungseinheit.

2. Ultraschallendoskop nach Anspruch 1, weiterhin umfassend:
mehrere variable Verzögerungsleitungseinheiten (DL₁₋₁ bis DL₁₅₋₅) mit ersten Anschlüssen, die an die zweite Gruppe von Verbindungen (L₁₋₁ bis L₁₅₋₅) angeschlossen sind; und
eine dritte Gruppe von Verbindungen (L1 bis L15), jeweils zum gegenseitigen Verbinden zweiter Anschlüsse der mehreren veränderlichen Verzögerungsleitungseinheiten, die an die mehreren Ultraschallwandler innerhalb der gleichen Reihe der mehreren zweidimensionalen Ultraschallwandlerfelder (TA1 bis TA12) angeschlossen sind, und zum elektrischen Verbinden eines Verbindungspunkts von ihnen mit einer externen Schaltung.

3. Ultraschallendoskopvorrichtung, umfassend:
ein Ultraschallendoskop gemäß Anspruch 1;
eine Sendeeinrichtung (14) zum Erzeugen von Treibersignalen und zum Ausgeben der Treibersignale zu den mehreren zweidimensionalen Ultraschallwandlerfeldern (TA1 bis TA12) über die zweite Gruppe von Verbindungen (EL₁₋₁ bis EL₁₅₋₅);
mehrere Teile einer Schalteinrichtung (SW1 bis SW12), die an die erste Gruppe von Verbindungen (G1 bis G12) und ein fixes Potential angeschlossen sind; und
eine Steuereinrichtung (12) zum Steuern, ob jede aus der ersten Gruppe von Verbindungen (G1 bis G12) an das fixe Potential angeschlossen ist oder nicht, indem Steuersignale an die mehreren Teile der Schalteinrichtung (SW1 bis SW12) geschickt werden, um aus den mehreren zweidimensionalen Ultraschallwandlerfeldern (TA1 bis TA12) ein zu verwendendes Feld auszuwählen, und zum Steuern von Verzögerungszeiten der Treibersignale in der Sendeeinrichtung (14) derart, daß das ausgewählte zweidimensionale Ultraschallwandlerfeld die dreidimensionale Abtastung innerhalb eines zugehörigen Bereichs ausführt.

4. Ultraschallendoskopvorrichtung, umfassend:
ein Ultraschallendoskop gemäß Anspruch 2;
eine Sendeeinrichtung (14) zum Erzeugen von Treibersignalen und zum Ausgeben der Treibersignale an die mehreren zweidimensionalen Ultraschallwandlerfeldern (TA1 bis TA12) über die dritte Gruppe von Verbindungen (L1 bis L15);
mehrere Teile einer Schalteinrichtung (SW1 bis SW12), die an die erste Gruppe von Verbindungen (G1 bis G12) und ein fixes Potential angeschlossen sind; und
eine Steuereinrichtung (12) zum Steuern, ob jede aus der ersten Gruppe von Verbindungen (G1 bis G12) an das fixe Potential angeschlossen ist oder nicht, indem Steuersignale an die mehreren Teile der Schalteinrichtung (SW1 bis SW12) gesendet werden, um unter den mehreren zweidimensionalen Ultraschallwandlerfeldern (TA1 bis TA12) ein zu verwendendes Feld auszuwählen, und zum Liefern von Verzögerungszeit-Steuersignalen zum Steuern der Verzögerungszeiten der Treibersignale an die mehreren variablen Verzögerunsgleitungseinheiten (DL₁₋₁ bis DL₁₅₋₅) derart, daß das ausgewählte zweidimensionale Ultraschallwandlerfeld die dreidimensionale Abtastung innerhalb eines zugehörigen Bereichs ausführt.

5. Ultraschallendoskopvorrichtung nach Anspruch 3, bei der die Steuereinrichtung (12) die Verzögerungszeiten der Treibersignale in der Sendeeinrichtung (14) derart steuert, daß die dreidimensionale Abtastung nach einem Sektoverfahren durchgeführt wird.

6. Ultraschallendoskopvorrichtung nach Anspruch 4, bei der die Steuereinrichtung (12) die Verzögerungszeit-Steuersignale zum Steuern der Verzögerungszeiten der Treibersignale an die mehreren variablen Verzögerungsleitungseinheiten (DL₁₋₁ bis DL₁₅₋₅) derart liefert, daß die dreidimensionale Abtastung nach einem Sektorverfahren durchgeführt wird.

7. Ultraschallendoskopvorrichtung nach Anspruch 3 oder 4, bei der die mehreren Teile der Schalteinrichtung (SW1 bis SW12) innerhalb des Ultraschallendoskops vorgesehen sind.

8. Ultraschallendoskopvorrichtung, umfassend:
ein Ultraschallendoskop gemäß Anspruch 1;
einen Adapter mit mehreren Teilen einer Schalteinrichtung (SW1 bis SW12), die mit der ersten Gruppe von Verbindungen (G1 bis G12) und einem fixen Potential verbunden sind; und
eine Ultraschall-Betrachtungsvorrichtung (3) mit einer Sendeeinrichtung (14) zum Erzeugen von Treibersignalen und zum Ausgeben der Treibersignale an die mehreren zweidimensionalen Ultraschallwandlerfelder (TA1 bis TA12) über die zweite Gruppe von Verbindungen (EL₁₋₁ bis EL₁₅₋₅), und mit einer Steuereinrichtung (12) zum Steuern, ob jede aus der ersten Gruppe von Verbindungen (G1 bis G12) mit dem fixen Potential verbunden ist oder nicht, indem Steuersignale an die mehreren Teile der Schalteinrichtung (SW1 bis SW12) geliefert werden, um aus den mehreren zweidimensionalen Ultraschallwandlerfeldern (TA1 bis TA12) ein zu verwendendes Feld auszuwählen, und zum Steuern der Verzögerungszeiten der Treibersignale in der Sendeeinrichtung (14) derart, daß das ausgewählte zweidimensionale Ultraschallwandlerfeld die dreidimensionale Abtastung innerhalb eines zugehörigen Bereichs durchführt.

9. Ultraschallendoskopvorrichtung, umfassend:
ein Ultraschallendoskop gemäß Anspruch 2; und
einen Adapter mit mehreren Teilen der Schalteinrichtung (SW1 bis SW12), die an die erste Gruppe von Verbindungen (G1 bis G12) und ein fixes Potential angeschlossen sind; und
eine Ultraschall-Betrachtungsvorrichtung (3) mit einer Sendeeinrichtung (14) zum Erzeugen von Treibersignalen und zum Ausgeben der Treibersignale an die mehreren zweidimensionalen Ultraschallwandlerfelder (TA1 bis TA12) über die dritte Gruppe von Verbindungen (L1 bis L15) und eine Steuereinrichtung (12) zum Steuern, ob jede aus der ersten Gruppe von Verbindungen (G1 bis G12) an das fixe Potential angeschlossen ist oder nicht, indem Steuersignale an die mehreren Teile der Schalteinrichtung (SW1 bis SW12) gesendet werden, um unter den mehreren zweidimensionalen Ultraschallwandlerfeldern (TA1 bis TA12) ein zu verwendendes Feld auszuwählen, und zum Liefern von Verzögerungszeit-Steuersignalen zum Steuern der Verzögerungszeiten der Treibersignale an die mehreren variablen Verzögerunsgleitungseinheiten (DL₁₋₁ bis DL₁₅₋₅) derart, daß das ausgewählte zweidimensionale Ultraschallwandlerfeld die dreidimensionale Abtastung innerhalb eines zugehörigen Bereichs ausführt.

## Revendications

1. Endoscope à ultrasons comprenant :
plusieurs ensembles de transducteurs à ultrasons en deux dimensions (TA1 à TA12) agencés en un demi-cercle ou selon une forme polygonale, comprenant chacun plusieurs transducteurs à ultrasons agencés en M rangées et N colonnes, où M et N sont des entiers non inférieurs à 2, afin d'effectuer une opération de balayage en trois dimensions ;
un premier groupe d'interconnexions (G1 à G12) destinées chacune à être reliées électriquement aux premières électrodes des différents transducteurs à ultrasons inclus dans lesdits ensembles de transducteurs à ultrasons en deux dimensions respectifs (TA1 à TA12) et à relier électriquement un point de connexion de ceux-ci à un circuit externe, **caractérisé par** :
un second groupe d'interconnexions (EL₁₋₁ à EL₁₅₋₅, L₁₋₁ à L₁₅₋₅) destinées chacune à être reliées électriquement aux secondes électrodes des différents transducteurs à ultrasons situés dans une même rangée et une même colonne dans lesdits ensembles de transducteurs à ultrasons en deux dimensions (TA1 à TA12) et à relier électriquement un point de connexion de ceux-ci à un circuit externe, directement ou par le biais d'une unité de ligne à retard variable.

2. Endoscope à ultrasons selon la revendication 1, comprenant en outre :
plusieurs unités de lignes à retard variable (DL₁₋₁ à DL₁₅₋₅) ayant des premières bornes reliées audit second groupe d'interconnexions (L₁₋₁ à L₁₅₋₅), respectivement ; et
un troisième groupe d'interconnexions (L1 à L15) chacune destinées à être électriquement reliées aux secondes bornes des différentes unités de lignes à retard variable reliées aux différents transducteurs à ultrasons situés dans une même rangée dans lesdits différents ensembles de transducteurs à ultrasons en deux dimensions (TA1 à TA12) et à relier électriquement un point de connexion de celles-ci à un circuit externe.

3. Appareil endoscopique à ultrasons comprenant :
un endoscope à ultrasons selon la revendication 1 ;
un moyen de transmission (14) destiné à générer des signaux d'entraînement et à transmettre les signaux d'entraînement auxdits différents ensembles de transducteurs à ultrasons en deux dimensions (TA1 à TA12) via ledit second groupe d'interconnexions (EL₁₋₁ à EL₁₅₋₅) ;
plusieurs moyens de commutation (SW1 à SW12) reliés audit premier groupe d'interconnexions (G1 à G12) et à un potentiel fixe ; et
un moyen de commande (12) destiné à contrôler le fait que chacune dudit premier groupe d'interconnexions (G1 à G12) soit reliée ou non au potentiel fixe en fournissant des signaux de commande auxdits moyens de commutation (SW1 à SW12) de façon à sélectionner celui à utiliser parmi lesdits ensembles de transducteurs à ultrasons en deux dimensions (TA1 à TA12), et à contrôler les quantités de retard des signaux d'entraînement dans ledit moyen de transmission (14) de telle sorte que l'ensemble de transducteurs à ultrasons en deux dimensions sélectionné effectue l'opération de balayage en trois dimensions dans une zone respective.

4. Appareil endoscopique à ultrasons comprenant :
un endoscope à ultrasons selon la revendication 2 ;
un moyen de transmission (14) destiné à générer des signaux d'entraînement et à transmettre les signaux d'entraînement auxdits différents ensembles de transducteurs à ultrasons en deux dimensions (TA1 à TA12) via ledit troisième groupe d'interconnexions (L1 à L15) ;
plusieurs moyens de commutation (SW1 à SW12) reliés audit premier groupe d'interconnexions (G1 à G12) et à un potentiel fixe ; et
un moyen de commande (12) destiné à contrôler le fait que chacune dudit premier groupe d'interconnexions (G1 à G12) soit reliée ou non au potentiel fixe en fournissant des signaux de commande auxdits moyens de commutation (SW1 à SW12) de façon à sélectionner celui à utiliser parmi lesdits différents ensembles de transducteurs à ultrasons en deux dimensions (TA1 à TA12), et à fournir des signaux de commande de quantité de retard permettant de contrôler les quantités de retard des signaux d'entraînement auxdites unités de lignes à retard variable (DL₁₋₁ à dDL₁₅₋₅) de telle sorte que l'ensemble de transducteurs à ultrasons en deux dimensions sélectionné effectue l'opération de balayage en trois dimensions dans une zone respective.

5. Appareil endoscopique à ultrasons selon la revendication 3, ledit moyen de commande (12) contrôlant les quantités de retard des signaux d'entraînement dans ledit moyen de transmission (14) de façon à effectuer l'opération de balayage en trois dimensions selon un procédé par secteurs.

6. Appareil endoscopique à ultrasons selon la revendication 4, ledit moyen de commande (12) fournissant les signaux de commande de quantité de retard permettant de contrôler les quantités de retard des signaux de commande auxdites différentes unités de lignes à retard variable (DL₁₋₁ à DL₁₅₋₅) de façon à effectuer l'opération de balayage en trois dimensions selon un procédé par secteurs.

7. Appareil endoscopique à ultrasons selon la revendication 3 ou 4, lesdits moyens de commutation (SW1 à SW12) étant prévus dans ledit endoscope à ultrasons.

8. Appareil endoscopique à ultrasons comprenant :
un endoscope à ultrasons selon la revendication 1 ;
un adaptateur comprenant plusieurs moyens de commutation (SW1 à SW12) reliés audit premier groupe d'interconnexions (G1 à G12) et à un potentiel fixe ; et
un appareil d'observation à ultrasons (3) comprenant un moyen de transmission (14) destiné à générer des signaux d'entraînement et à transmettre les signaux d'entraînement auxdits différents ensembles de transducteurs à ultrasons en deux dimensions (TA1 à TA12) via ledit second groupe d'interconnexions (EL₁₋₁ à EL₁₅₋₅), et un moyen de commande (12) destiné à contrôler le fait que chacune dudit premier groupe d'interconnexions (G1 à G12) soit reliée ou non au potentiel fixe en fournissant des signaux de commande auxdits différents moyens de commutation (SW1 à SW12) de façon à sélectionner celui à utiliser parmi lesdits différents ensembles de transducteurs à ultrasons en deux dimensions (TA1 à TA12), et à contrôler les quantités de retard des signaux d'entraînement dans ledit moyen de transmission (14) de telle sorte que l'ensemble de transducteurs à ultrasons en deux dimensions sélectionné effectue l'opération de balayage en trois dimensions dans une zone respective.

9. Appareil endoscopique à ultrasons comprenant :
un endoscope à ultrasons selon la revendication 2 ; et
un adaptateur comprenant plusieurs moyens de commutation (SW1 à SW12) reliés audit premier groupe d'interconnexions (G1 à G12) et à un potentiel fixe ; et
un appareil d'observation à ultrasons (3) comprenant un moyen de transmission (14) destiné à générer des signaux d'entraînement et à transmettre les signaux d'entraînement auxdits différents ensembles de transducteurs à ultrasons en deux dimensions (TA1 à TA12) via ledit troisième groupe d'interconnexions (L1 à L15), et un moyen de commande (12) destiné à contrôler le fait que chacune dudit premier groupe d'interconnexions (G1 à G12) soit reliée ou non au potentiel fixe en fournissant des signaux de commande auxdits différents moyens de commutation (SW1 à SW12) de façon à sélectionner celui à utiliser parmi lesdits différents ensembles de transducteurs à ultrasons en deux dimensions (TA1 à TA12), et à fournir des signaux de commande de quantité de retard permettant de contrôler les quantités de retard des signaux d'entraînement auxdites différentes unités de lignes à retard variable (DL₁₋₁ à DL₁₅₋₅) de telle sorte que l'ensemble de transducteurs à ultrasons en deux dimensions sélectionné effectue l'opération de balayage en trois dimensions dans une zone respective.
